# EUROPÄISCHE PATENTSCHRIFT

(11) **EP 0 496 110 B1**
(45) Veröffentlichungstag und Bekanntmachung des Hinweises auf die Patenterteilung: **05.03.1997**
(21) Anmeldenummer: 91122245.3
(22) Anmeldetag: 27.12.1991
(51) Int. Cl.: A61B 1/00, A61B 1/307, A61B 1/12, A61B 17/32

(54) **Endoskop zur transurethralen Operation**
Endoscope for transurethral operation
Endoscope pour opération transurétrale

(30) Priorität: 19.01.1991 DE 4101472
(43) Veröffentlichungstag der Anmeldung: 29.07.1992
(73) Patentinhaber: OLYMPUS WINTER & IBE GmbH, 22045 Hamburg (DE)
(72) Erfinder: Faul, Peter, Prof. Dr., D-8940 Memmingen (DE); Hluchy, Heinz, D-2000 Hamburg 63 (DE); Schlüter, Armin, D-2000 Hamburg 26 (DE)
(74) Vertreter: Schaefer, Konrad, Dipl.-Phys.

(56) Entgegenhaltungen:
- EP-A- 0 131 971
- EP-A- 0 199 848
- EP-A- 0 225 690
- US-A- 2 708 437
- US-A- 4 790 295

## Beschreibung

Die Erfindung betrifft ein Endoskop der im Oberbegriff des Anspruches 1 genannten Art.

Endoskope für die transurethrale Operation werden mit ihrem Außenschaft in die Urethra des Patienten gelegt. Mit der Optik kann dann das Operationsgebiet betrachtet werden. Mit dem Operationsinstrument, das beispielsweise eine Schere, Zange oder üblicherweise eine hochfrequenzbeaufschlagte Schneidelektrode ist, kann operiert, z.B. geschnitten werden. Dazu sind am Hauptkörper des Endoskopes Betätigungseinrichtungen vorgesehen, mit denen das Operationsinstrument betätigt, beispielsweise in Achsrichtung hin- und hergeschoben werden kann. Üblicherweise sind am Endoskop Spülwasseranschlüsse vorgesehen, mit denen das Operationsgebiet zur Schaffung besserer Sichtverhältnisse freigespült werden kann.

Im Falle von Endoskopen mit Pistolengriff am Hauptkörper sind Dreheinrichtungen bekannt, mit denen der Pistolengriff und Teile des Hauptkörpers gegenüber den übrigen Teilen des Endoskopes verdrehbar sind. Dabei bleiben aber das Operationsinstrument und der Außenschaft gegeneinander stets drehfest.

Beim Operieren in der Blase, insbesondere beim Resizieren in der Prostata, ist es erforderlich, häufig die Drehorientierung des Operationsinstrumentes zum Operationsgebiet zu ändern. So muß beispielsweise beim Operieren in der Prostata abwechselnd rechts, links, oben und unten resiziert werden. Bei bekannten Endoskopen der eingangs genannten Art, bei denen das Operationsinstrument gegenüber dem Außenschaft feststeht, ist es daher erforderlich, ständig den Außenschaft in der Urethra zu drehen. Durch Reibung der Schaftaußenwand mit der Wand der Urethra kommt es aber zu Irritationen der empfindlichen Schleimhaut der Urethra und gegebenenfalls zu Verletzungen.

Aus der EP 0 199 848 A1 ist ein gattungsgemäßes Endoskop beschrieben, bei dem auch der Zuflußkanal, getrennt vom Abflußkanal, im Zwischenraum zwischen dem Innenschaft und dem Außenschaft vorgesehen ist. Eine Konstruktion mit Zuflußkanal im Innenschaft und Abflußkanal zwischen Innenschaft und Außenschaft, wie sie bei den Endoskopen zur transurethralen Operation üblich ist, ist bei dieser Konstruktion nicht möglich. Vorteilhaft bei dieser Konstruktion ist aber die Tatsache, daß bei während der Operation erforderlichen Verdrehungen des Hauptkörpers mit der Optik der Außenschaft drehfest im Operationskanal bzw. bei Verwendung zur transurethralen Operation in der Urethra liegen kann, ohne deren Wände durch ständiges Verdrehen zu beschädigen. Dabei bleiben mit dem Außenschaft auch die beiden Spülanschlüsse drehfest liegen, so daß der Operateur nicht durch ständiges Verdrehen der an den Anschlüssen vorgesehenen Zu- und Abfuhrschläuche behindert wird.

Aus der US-PS 4 790 295 ist ein Endoskop mit einem Schaft vorgesehen, in dem eine Optik und ein Arbeitskanal zum Einführen eines Operationsinstrumentes angeordnet sind.

Die EP 0 225 690 A1 zeigt ein Ultraschallendoskop mit einem Außenschaft und einem Innenschaft, die beide drehfest mit dem Hauptkörper verbunden sind. Der Zwischenraum zwischen dem In nenschaft und dem Außenschaft dient hier als Zuflußkanal, während der Innenraum des Innenschaftes als Abflußkanal dient. Nachteilig bei dieser Konstruktion ist die drehfete Verbindung des Außenschaftes mit dem gesamten Endoskop, so daß bei Verdrehungen des Endoskopes stets der Außenschaft gegenüber dem Körpergewebe mitgedreht wird.

Aus der EP 0 131 971 A3 ist ein flexibles Endoskop bekannt, bei dem die Verriegelung seines Anschlußkabels an einem Anschlußgerät drehbar ausgebildet ist.

Die Aufgabe der vorliegenden Erfindung besteht darin, ein gattungsgemäßes Endoskop für die speziellen Bedürfnisse der transurethralen Operation mit ausreichender Spülwirkung auszubilden.

Diese Aufgabe wird erfindungsgemäß mit den Merkmalen des Kennzeichnungsteiles des Auspruchs 1 gelöst.

Bei diesem Endoskop ist, wie dies für die transurethrale Operation von großem Vorteil ist, der Zuflußkanal im Innenschaft vorgesehen, während der Abflußkanal zwischen den Schäften ausgebildet ist. Dabei bleibt der Vorteil der gattungsgemäßen Konstruktion erhalten, wonach der Außenschaft bei Verdrehung des Endoskopes zusammen mit den Anschlußschläuchen drehfest zum Patienten liegenbleibt. Bei den sehr häufig erforderlichen Verdrehungen des Endoskopes wird die Urethra nicht durch Verdrehungen des Außenschaftes beschädigt und es wird der Operateur nicht durch Schlauchverdrehungen behindert.

Eine Ausgestaltung nach Anspruch 2 hat den Vorteil, daß bei Bedarf der Innenschaft für sich allein als Schaft zur intermittierenden Spülung mit drehbarem Spülanschluß genutzt werden kann. Wird der Außenschaft an den drehbaren Spülanschluß des Innenschaftes fest angekoppelt, ist der Zuflußanschluß wie der Abflußanschluß in Relation zum Außenschaft fixiert und der Innenschaft drehbar zum Außenschaft.

Da es zur Reinigung und Sterilisation günstig ist, wenn Außenschaft und Innenschaft voneinander getrennt werden, ist üblicherweise eine Verriegelung zwischen Innen- und Außenschaft vorgesehen. In vorteilhafter Weise wird entsprechend Anspruch 3 die Verriegelung gleichzeitig als Drehlager für den Innenschaft ausgebildet. Diese Anordnung ist raumsparend und handhabungsfreundlich.

Bei dem drehbaren Spülanschluß eines bekannten Schaftes mit intermittierender Spülung (ohne Außenschaft) wird ein sogenannter O-Ring als Dichtung verwendet. Würde man einen solchen O-Ring beim erfindungsgemäßen Endoskop zur Abdichtung zwischen Außen- und Innenschaft verwenden, so wäre die Reibkraft zwischen Innenschaft und Außenschaft größer als die Reibkraft zwischen Außenschaft und Harnröhre, so daß ein unerwünschtes Mitdrehen und somit Reibung zwischen Außenschaft und Harnröhre auftreten könnte. Entsprechend Anspruch 4 wird deshalb vorteilhaft eine reibungsarme Lippendichtung zur Abdichtung zwischen Innen- und Außenschaft angeordnet.

In der Zeichnung ist die Erfindung beispielsweise und schematisch dargestellt. Es zeigen:
- Fig. 1: eine erste Ausführungsform eines erfindungsgemäßen Endoskopes, teilweise im Schnitt,
- Fig. 2: das Endoskop der Fig. 1 entlang der Linie A - B geschnitten und
- Fig. 3: eine erfindungsgemäße Variante mit drehbarem Zuflußanschluß am Innenschaft in Seitenansicht.

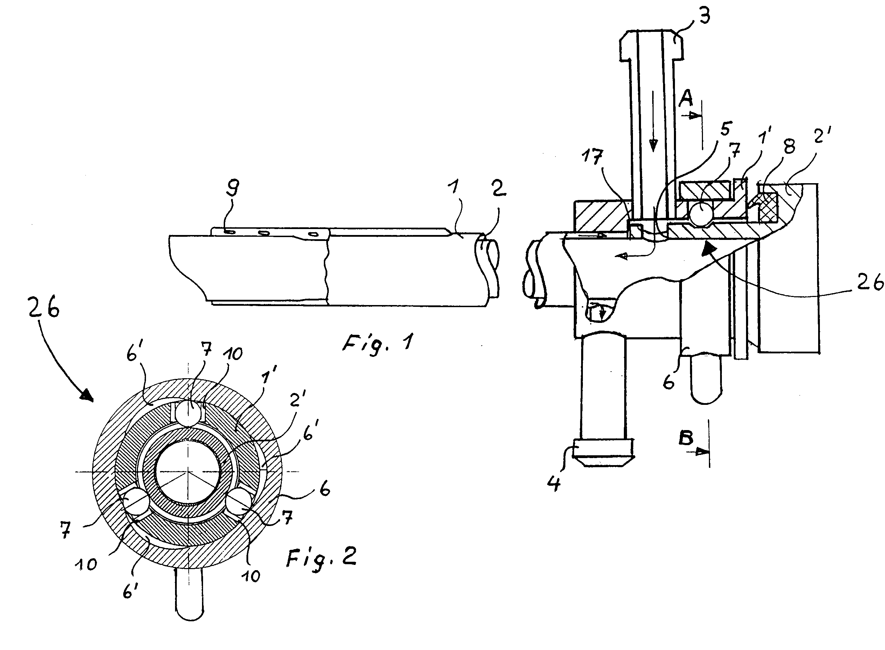

In Fig. 1 ist eine erste Ausführungsform des erfindungsgemäßen Endoskopes - ohne Optik und Instrumententräger - dargestellt, bestehend aus einem Außenschaft 1 und einem eingeschobenen Innenschaft 2. Der Außenschaft 1 verdickt sich an seinem proximalen Ende zu einem Verschlußkörper 1' und der Innenschaft 2 zu einem Verschlußteil 2'. Ein Zuflußanschluß 3 und ein Abflußanschluß 4 befinden sich fest am Verschlußkörper 1'. Zur Operation wird über den Zuflußanschluß 3 Spülflüssigkeit zugeführt. Über eine bzw. mehrere Bohrungen 5 in einem Ringkanal des Verschlußteiles 2' gelangt die Spülflüssigkeit in den Kanal des Innenschaftes 2 und kann aus dessem distalen Ende ausströmen. Über Bohrungen 9 und über einen Kanal, der durch den Zwischenraum zwischen Außenschaft 1 und dem Innenschaft 2 gebildet wird, wird die Spülflüssigkeit über den Abflußanschluß 4 wieder abgesaugt.

Mit dem Verschlußteil 2' kann die in Fig. 1 dargestellte Schaftanordnung am nicht dargestellten Hauptkörper eines Endoskopes befestigt werden.

Das Verschlußteil 2'des Innenschaftes 2 ist über eine Verriegelung 26 mit dem Verschlußkörper 1' des Außenschaftes 1 drehbar verbunden. Der Verschlußkörper 1' dient dabei als Käfig eines Kugellagers, dessen Kugeln 7 in einer Ringnut des Verschlußteiles 2' laufen, wodurch eine Bewegung in axialer Richtung zwischen Innenschaft 2 und Außenschaft 1 vermieden wird. Zur Abdichtung zwischen Verschlußkörper 1' und Verschlußteil 2' dient eine reibungsarme Lippendichtung 8.

Zur Resektion kann der Innenschaft 2 einschließlich hier nicht dargestelltem Operationsinstrument und nicht dargestellter Optik um seine Längsachse gedreht werden, während der Außenschaft 1 in vorteilhafter Weise in seiner Position verbleiben kann, so daß eine Reibung zwischen Außenschaft und Harnröhre, verursacht durch die Rotation des Endoskopes, praktisch nicht auftritt. Durch die Lippendichtung, deren Reibkraft geringer ist als die Reibkraft zwischen Außenschaft und Harnröhre, wird die Rotation des Außenschaftes 1 - auch wenn dieser nicht von der Hand des Operateurs in seiner Stellung fixiert wird - vermieden. Da der Außenschaft 1 nicht mehr rotiert, entfällt in weiterer vorteilhafter Weise zugleich das unerwünschte Verwinden der an ihm angeschlossenen Schläuche für Zu- und Abfluß der Spülflüssigkeit. Zur Abdichtung zwischen Außenschaft 1 und Innenschaft 2 ist eine Engstelle 17 vorgesehen, wodurch ein Strömungskurzschluß zwischen Zulaufkanal und Ablaufkanal klein gehalten wird. Soll der Strömungskurzschluß ganz vermieden werden, ist hier eine Dichtung, vorzugsweise eine reibungsarme Lippendichtung, vorzusehen.

Der in Fig. 2 dargestellte Schnitt zeigt die Verriegelung 26 des Endoskopes der Fig. 1 in verriegelter Stellung. Die Verriegelung 26 fixiert Außenschaft 1 und Innenschaft 2 in ihrer axialen Position zueinander und dient gleichzeitig als Drehlager. Die Kugeln 7 werden durch eine Kugelhalterung 10 im Verschlußkörper 1', der somit als Käfig eines Kugellagers dient, gehalten. In verriegelter Stellung laufen die Kugeln 7 in einer Ringnut des Verschlußteiles 2' des Innenschaftes 2. Durch Drehung des Verriegelungsringes 6 in Uhrzeigerrichtung werden die Kugeln 7 in axialer Richtung durch seine Ausbuchtungen 6' freigegeben, und der Innenschaft 2 kann aus dem Außenschaft 1 herausgezogen werden.

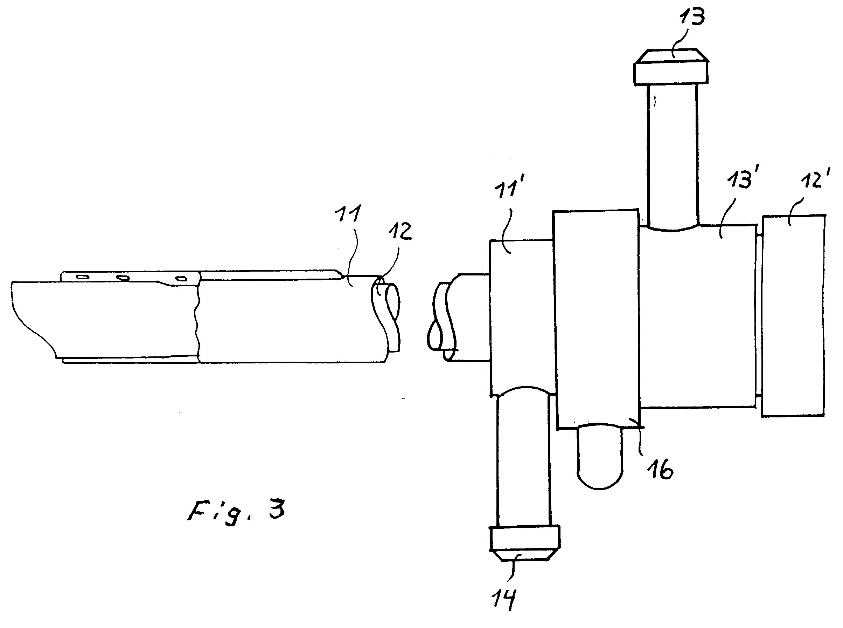

In Fig. 3 ist ein Endoskop, bestehend aus einem Außenschaft 11 mit festem Abflußanschluß 14 und einem Innenschaft 12 mit Zuflußanschluß 13 an Drehring 13' als eine erfindungsgemäße Variante dargestellt. Der Außenschaft 11 verdickt sich an seinem proximalen Ende zu einem Verschlußkörper 11', an dem sich der Abflußanschluß 14 und ein Verriegelungsring 16 befinden. Der Innenschaft 12 verdickt sich an seinem proximalen Ende zu einem Verschlußteil 12', an dem der Zuflußanschluß 13 drehbar durch den Drehring 13' sich befindet. Der Innenschaft 12 ist in den Außenschaft 11 eingeschoben und der Drehring 13' über den Verriegelungsring 16 mit dem Verschlußkörper 11' des Außenschaftes 11 fest verriegelt.

Zur Resektion kann der Innenschaft 12 (mit Verschlußteil 12') um seine Achse gedreht werden, während Außenschaft 11 mit Abflußanschluß 14 und angekoppeltem Drehring 13' und Zuflußanschluß 13 in seiner Position verbleiben kann, so daß auch hier die Rotationsreibung zwischen Harnröhre und Außenschaft 11 und das nachteilige Verwinden der angeschlossenen Schläuche ebenfalls wegfallen. Gegenüber der Variante von Fig. 1 besteht der zusätzliche Vorteil, daß das Endoskop ohne Außenschaft 11 bei Bedarf als Endoskop mit intermittierender Spülung und drehbarem Zuflußanschluß 13 eingesetzt werden kann.

## Patentansprüche

1. Endoskop zur transurethralen Operation mit einem rohrförmigen Außenschaft (1, 11), mit einem innerhalb des Außenschaftes angeordneten, eine Optik rohrförmig umgebenden, dieser gegenüber drehfest stehenden Innenschaft (2, 12), wobei der Innendurchmesser des Außenschaftes größer als der Außendurchmesser des Innenschaftes ist, wobei der Außenschaft gegenüber den übrigen Teilen des Endoskopes drehbar angeordnet ist, wobei die Schäfte (1, 11; 2, 12) proximalseitig zueinander abgedichtet sind, und wobei ein an den einen Abflußkanal bildenden Zwischenraum zwischen den Schäften (1, 2; 11, 12) angeschlossener Abflußanschluß (4, 14) und ein Zuflußanschluß (3, 13) vorgesehen sind, die beide drehfest an dem Außenschaft (1, 11) in dessen proximalem Endbereich angeordnet sind, **dadurch gekennzeichnet,** daß der Außenschaft (1, 11) als Verschlußkörper (1'; 11', 13') endet und der Innenschaft (2, 12) als Verschlußteil (2', 12') endet, daß der Verschlußkörper und das Verschlußteil in Axialrichtung fixiert, aufeinander drehbar gelagert sind und zwischen sich einen Ringkanal begrenzen, der in proximaler und distaler Richtung durch Drehdichtungen (8, 17) abgeschlossen ist, und daß der Zuflußanschluß (3, 13) in den Ringkanal mündet, der seinerseits über wenigstens eine Bohrung (5) im Innenschaft (2, 12) mit dessen als Zuflußkanal ausgebildetem Innenraum in Verbindung steht.

2. Endoskop nach Anspruch 1, **dadurch gekennzeichnet,** daß der Zuflußanschluß (13) drehbar am Innenschaft (12) angeordnet ist und der Außenschaft (11) mit dem Zuflußanschluß (13) lösbar gekoppelt ist.

3. Endoskop nach einem der vorhergehenden Ansprüche mit Verriegelung zwischen Innen- und Außenschaft, **dadurch gekennzeichnet,** daß die Verriegelung (26) gleichzeitig als Drehlager ausgebildet ist.

4. Endoskop nach einem der vorhergehenden Ansprüche, **dadurch gekennzeichnet,** daß wenigstens eine Lippendichtung (8) zur Abdichtung zwischen Innen- (2, 12) und Außenschaft (1, 11) angeordnet ist.

## Claims

1. Endoscope for transurethral operations with a tubular outer shaft (1, 11), with an inner shaft (2, 12) which is arranged within the outer shaft, surrounds an optical system in the manner of a tube and is rotationally fixed with respect to it, the inner diameter of the outer shaft being greater than the outer diameter of the inner shaft, the outer shaft being rotatably arranged with respect to the other components of the endoscope, the shafts (1, 11; 2, 12) being sealed with respect to one another at the proximal end and a discharge connection (4, 14), connected to the space between the shaft (1, 2; 11, 12) constituting a discharge passage, and a supply connection (3, 13) being provided, both of which are rotationally fixedly arranged on the outer shaft (1, 11) in its proximal end region, characterised in that the outer shaft (1, 11) terminates in a closure body (1'; 11', 13') and the inner shaft (2, 12) terminates in a closure member (2', 12'), that the closure body and the closure member are fixed in the axial direction and are mounted to be rotatable on one another and define between them an annular passage which is closed in the proximal and distal direction by rotary seals (8, 17) and that the supply connection (3, 13) discharges into the annular passage which for its part communicates via at least one bore (5) in the inner shaft (2, 12) with its internal space, which is constructed as a supply passage.

2. Endoscope as claimed in Claim 1, characterised in that the supply connection (13) is rotatably arranged on the inner shaft (12) and the outer shaft (11) is releasably coupled to the supply connection (13).

3. Endoscope as claimed in one of the preceding claims with a lock between the inner and outer shafts, characterised in that the lock (26) is constructed at the same time as a rotary bearing.

4. Endoscope as claimed in one of the preceding claims, characterised in that at least one lip seal (8) is arranged to form a seal between the inner (2, 12) and outer (1, 11) shafts.

## Revendications

1. Endoscope pour opération transurétrale muni d'une gaine extérieure tubulaire (1, 11) avec, à l'intérieur de celle-ci, une gaine intérieure (2, 12) entourant tubulairement une optique sans possibilité de rotation par rapport à celle-ci, le diamètre intérieur de la gaine extérieure étant supérieur au diamètre extérieur de la gaine intérieure et la gaine extérieure pouvant tourner par rapport aux autres parties de l'endoscope, les gaines (1, 11; 2, 12) étant étanchéifiées l'une par rapport à l'autre du côté proximal et l'espace intermédiaire entre les gaines (1, 2; 11, 12) formant un canal d'évacuation sur lequel sont agencés un raccord d'évacuation (4, 14) et un raccord d'adduction (3, 13), tous deux fixés sans possibilité de rotation à la gaine extérieure (1, 11) dans la zone de son extrémité proximale, **caractérisé en ce que** la gaine extérieure (1, 11) et la gaine intérieure (2, 12) se terminent respectivement en un corps d'obturation (1'; 11', 13') et une pièce d'obturation (2', 12'), et en ce que le corps d'obturation et la pièce d'obturation sont fixés dans le sens axial et logés l'un sur l'autre de façon à pouvoir pivoter et à délimiter entre eux un canal annulaire obturé dans le sens proximal et dans le sens distal par des joints (8, 17) pour pièces en rotation, et en ce que le raccord d'adduction (3, 13) débouche dans le canal annulaire qui communique par au moins un alésage (5) de la gaine intérieure (2, 12) avec l'espace intérieur de celle-ci formant le canal d'adduction.

2. Endoscope selon la revendication 1, **caractérisé en ce que** le raccord d'adduction (13) est monté sur la gaine intérieure (12) de façon à pouvoir pivoter et en ce que la gaine extérieure (11) est coupée au raccord d'adduction (13) par un accouplement amovible.

3. Endoscope selon l'une des revendications précédentes muni d'un dispositif de verrouillage entre la gaine intérieure et la gaine extérieure, caractérisé en ce que le dispositif de verrouillage (26) est conçu de façon à servir également de coussinet de pivotement.

4. Endoscope selon l'une des revendications précédentes, **caractérisé en ce qu**'il est muni d'au moins un joint à lèvre (8) pour assurer l'étanchéification entre la gaine intérieure (2, 12) et la gaine extérieure (1, 11).
